# EUROPÄISCHE PATENTANMELDUNG

(11) **EP 1 795 220 A1**
(43) Veröffentlichungstag der Anmeldung: **13.06.2007**
(21) Anmeldenummer: 06023871.4
(22) Anmeldetag: 17.11.2006
(51) Int. Cl.: A61M 5/32

(54) **Spritze mit Verschluss**

(30) Priorität: 30.11.2005 DE 102005058133
(71) Anmelder: Schott AG, 55122 Mainz (DE)
(72) Erfinder: Mösli, Thomas, 9014 St. Gallen (CH); Wolbring, Peter, Dr., 66606 St. Wendel (DE)
(74) Vertreter: Gahlert, Stefan

(57) **Zusammenfassung**

Es wird eine Spritze (10) mit einem Verschluss (12) angegeben, mit einer aus einem Spritzenkörper (26) hervorstehenden Kanüle (28), deren Spitze (30) in einer Schließstellung der Spritze (10) von einer Verschlusskappe (34) umschlossen ist, und mit einem Dichtungselement (14) zur Abdichtung der Kanüle (28) in der Schließstellung, das einen ringförmigen, elastisch verformbaren Bereich (18) aufweist, der mittels der Verschlusskappe (34) an die Außenoberfläche der Kanüle (28) dichtend anpressbar ist (Fig. 2).

## Beschreibung

Die Erfindung betrifft eine Spritze mit einem Verschluss, mit einer aus einem Spritzenkörper hervorstehenden Kanüle, deren Spitze in einer Schließstellung der Spritze von einer Verschlusskappe umschlossen ist, und mit einem Dichtungselement zur Abdichtung der Kanüle in der Schließstellung.

Die Erfindung betrifft ferner einen Verschluss für eine Spritze, die eine aus einem Spritzenkörper hervorstehende Kanüle aufweist, wobei der Verschluss zur Montage auf einem Ende des Spritzenkörpers ausgebildet ist und eine Verschlusskappe aufweist, die eine Spitze der Kanüle in einer Schließstellung umschließt, und mit einem Dichtungselement zur Abdichtung der Kanüle in der Schließstellung.

Eine derartige Spritze mit einem derartigen Verschluss ist bspw. aus der EP 0 873 758 B1 bekannt.

Es handelt sich hierbei um eine Einmalspritze mit einer im Spritzenkörper fest aufgenommenen Injektionsnadel und mit einer Verschlusskappe zum Schutz der Injektionsnadel. Um eine Abdichtung der vorgefüllten Spritze zu gewährleisten, ist die Injektionsnadel in ein Dichtungselement eingestochen, das von der Verschlusskappe umschlossen ist.

Ähnliche Spritzen sind aus der US 6,719,732 B2 und aus der US 2005/0038391 A1 bekannt.

Die im Stand der Technik bekannten Spritzen werden mit flüssigen Substanzen befüllt und durch eine Kappe verschlossen, die man im internationalen Sprachgebrauch meist als "needle shield" bezeichnet. Sie besteht in der Regel aus einem gummielastischen Material, meist einem mineralgefüllten Synthesekautschuk mit einer Shore-Härte zwischen 40 A und 60 A. Um diese Verschlusskappen mechanisch stabiler zu machen, werden sie oft in ein festes Gehäuse eingebaut. Eine derartige Ausführungsform bezeichnet man dann als "rigid needle shield". Spritzen und Verschlüsse der eingangs genannten Art erfüllen grundsätzlich zwei wesentliche Funktionen. Zum einen verdecken sie die Spitze der in den Spritzenkörper eingebauten Kanüle, zum anderen verschließen sie distal den Innenraum des Spritzenkörpers in einer flüssigkeitsdichten Weise. Dies geschieht auf einfache Weise dadurch, dass die Kanülenspitze in das weiche Material der Schutzkappe eingestochen wird.

Diese Art der Abdichtung ist mit erheblichen Nachteilen für die Spritze und letztlich für die Patienten verbunden, die mit dieser Spritze behandelt werden. Insbesondere dünnwandige Kanülen mit geringen Außendurchmessern, wie sie heutzutage für vorgefüllte Spritzen meist verwendet werden, besitzen einen mechanisch äußerst empfindlichen Schliff. Je filigraner die Kanüle, desto anfälliger ist der Schliff für Beschädigungen. Eine Kanüle, die einmal in ein beliebiges Medium eingestochen wurde, kann bereits einen Teil ihrer Schärfe eingebüßt haben. In manchen Fällen kann es selbst beim Einstich in sehr weiche Medien zu einer häkelnadelartigen Deformation der Kanülenspitze kommen. Die Verwendung nicht optimal scharfer und an der Spitze deformierter Kanülen führt jedoch beim Patienten zu einer erheblichen Erhöhung des Punktionsschmerzes. Auch besteht die Gefahr, dass durch verformte Kanülenschliffe Blutungen, Hämatome oder sogar Abszesse entstehen. Ferner ist es seit Langem bekannt, dass beim Einstechen von Kanülen in gummielastische Materialien Partikel ausgestanzt werden, die im schlimmsten Fall die Kanüle verstopfen und die Injektion des Medikaments verhindern können. Bleiben sie dagegen beweglich, können sie mit der Injektion in den Patienten gespült werden.

Der Erfindung liegt somit die Aufgabe zu Grunde, eine verbesserte Spritze und einen verbesserten Verschluss für eine Spritze zu schaffen, womit ein sicherer Verschluss einer vorgefüllten Spritze ermöglicht ist, ohne dass die Spitze der Kanüle in ein Dichtungsmaterial eingestochen wird.

Diese Aufgabe wird erfindungsgemäß bei einer Spritze gemäß der eingangs genannten Art dadurch gelöst, dass das Dichtungselement einen ringförmigen, elastisch verformbaren Bereich aufweist, der mittels der Verschlusskappe an die Außenoberfläche der Kanüle dichtend anpressbar ist.

Die Aufgabe der Erfindung wird ferner bei einem Verschluss der eingangs genannten Art dadurch gelöst, dass das Dichtungselement einen ringförmigen, elastisch verformbaren Bereich aufweist, der mittels der Verschlusskappe an die Außenoberfläche der Kanüle dichtend anpressbar ist.

Die Aufgabe der Erfindung wird auf diese Weise vollkommen gelöst.

Erfindungsgemäß wird nämlich jegliches Einstechen der Spitze der Kanüle in ein Dichtungselement vermieden. Vielmehr erfolgt eine flüssigkeitsdichte Abdichtung der Kanüle am distalen Ende dadurch, dass der ringförmige, elastisch verformbare Bereich in der Schließstellung dichtend an die Außenoberfläche der Kanüle anpressbar ist. Auf diese Weise können die im Stand der Technik vorhandenen Nachteile vermieden werden. Insbesondere kann jegliche Berührung der Spitze der Kanüle mit dem Dichtungsmaterial vermieden werden. So kann keine Deformation der Spitze erfolgen. Gleichfalls wird ein Stanzeffekt vermieden, so dass kein durch Einstechen der Kanüle in ein Dichtungsmaterial ausgestanztes Fremdmaterial zu einer Kontamination beitragen kann.

In einer vorteilhaften Weiterbildung der Erfindung weist das Dichtungselement einen Hohlraum auf, in dem die Spitze der Kanüle in der Schließstellung vorzugsweise berührungsfrei aufgenommen ist.

Auf diese Weise wird die Gefahr einer möglichen Kontamination der Spritze im Bereich der Spitze der Kanüle weiter verringert.

Gemäß einer weiteren Ausgestaltung der Erfindung ist das Dichtungselement über den ringförmigen Bereich mit einem Druckelement verbunden, wobei der ringförmige Bereich durch eine Relativbewegung zwischen Dichtelement und Druckelement auf die Außenoberfläche der Kanüle anpressbar ist.

Auf diese Weise kann eine saubere Abdichtung der Spritze nach einem Füllvorgang gewährleistet werden, indem nach Beendigung eines Füllvorgangs der Spritze das Dichtelement und das Druckelement relativ zueinander bewegt werden, um den ringförmigen Bereich auf die Außenoberfläche der Kanüle anzupressen.

Gemäß einer weiteren Ausgestaltung der Erfindung ist zumindest das Dichtungselement von der Verschlusskappe zumindest teilweise umschlossen und mittels der Verschlusskappe relativ zum Druckelement bewegbar.

So kann durch eine Bewegung der Verschlusskappe relativ zum Druckelement das Dichtungselement durch die Verschlusskappe bewegt werden, um eine Abdichtung des ringförmigen Bereiches zu erreichen.

Gemäß einer weiteren Ausgestaltung der Erfindung sind das Dichtelement und das Druckelement axial zueinander beweglich.

Zusätzlich oder alternativ können das Dichtelement und das Druckelement gegeneinander verdrehbar sein.

So kann durch eine Axialbewegung oder durch eine Drehbewegung zwischen Dichtungselement und Druckelement eine Anpressung des ringförmigen Bereiches auf die Außenoberfläche der Kanüle erreicht werden.

Gemäß einer weiteren Ausgestaltung der Erfindung ist die Verschlusskappe in der Schließstellung verrastbar.

Dies ist eine besonders einfache Möglichkeit, um die Verschlusskappe in der Schließstellung in befülltem Zustand der Spritze gegen ein unbeabsichtigtes Öffnen zu sichern.

Gemäß einer weiteren Ausgestaltung der Erfindung ist am distalen Ende des Spritzenkörpers ein Wulst vorgesehen, an dem die Verschlusskappe oder das Druckelement in der Schließstellung befestigt ist.

Auf diese Weise ist eine einfache Befestigung der Verschlusskappe am Spritzenkörper ermöglicht.

Gemäß einer weiteren Ausgestaltung der Erfindung ist die Verschlusskappe mittels eines Gewindes, vorzugsweise mittels eines Luer-Lock-Gewindes, am Spritzenkörper festlegbar.

Auch auf diese weise kann eine einfache Sicherung der Verschlusskappe in der Schließstellung erreicht werden.

Gemäß einer weiteren Ausgestaltung der Erfindung ist ein Indikatorelement vorgesehen, das eine erstmalige Bewegung der Verschlusskappe aus der Schließstellung anzeigt.

So kann eine vormalige Betätigung der Spritze optisch erkennbar gemacht werden, um sicherzustellen, dass nur eine Spritze verwendet wird, bei der sich die Verschlusskappe noch in der Schließstellung mit der Originalbefüllung befindet.

Gemäß einer weiteren Ausgestaltung der Erfindung weist die Verschlusskappe eine Schnappverbindung mit Bügelelementen auf, die in der Schließstellung den ringförmigen Bereich auf die Außenoberfläche der Kanüle pressen.

Auf diese Weise wird die Abdichtung der Kanüle weiter verbessert.

In zusätzlicher Weiterbildung dieser Ausgestaltung ist die Schnappverbindung als nicht zerstörungsfrei lösbare Schnappverbindung ausgebildet, bei der eine erstmalige Bewegung der Verschlusskappe aus der Schließstellung optisch erkennbar ist.

Auf diese Weise kann die optische Anzeige einer erstmaligen Bewegung der Verschlusskappe aus der Schließstellung mit einfachen Mitteln verwirklicht werden.

Gemäß einer weiteren Ausgestaltung der Erfindung bestehen das Dichtungselement und das Druckelement aus demselben Material und sind einstückig ausgebildet.

Auf diese Weise kann eine Durchmesserverringerung im ringförmigen Bereich zwecks Abdichtung der Kanüle an der Außenoberfläche durch eine Relativbewegung zwischen Dichtungselement und Druckelement auf besonders einfache Weise erzielt werden.

Es versteht sich, dass die vorstehend genannten und die nachstehend noch zu erläuternden Merkmale der Erfindung nicht nur in der jeweils angegebenen Kombination, sondern auch in anderen Kombinationen oder in Alleinstellung verwendbar sind, ohne den Rahmen der Erfindung zu verlassen.

Weitere Merkmale und Vorteile der Erfindung ergeben sich aus der nachfolgenden Beschreibung bevorzugter Ausführungsbeispiele unter Bezugnahme auf die Zeichnung. Es zeigen:
- Fig. 1: einen Längsschnitt durch einen Einsatz mit einem Dichtungselement und einem Druckelement, der für einen erfindungsgemäßen Verschluss verwendet werden kann;
- Fig. 2: eine praktische Anwendung des Einsatzes gemäß Fig. 1 bei einer ersten Ausführung eines erfindungsgemäßen Verschlusses am Beispiel einer Glasspritze mit eingeklebter Kanüle, in längsgeschnittener Darstellung;
- Fig. 3: eine Abwandlung der Ausführung gemäß Fig. 2 in einer Ausgestaltung als Kunststoffspritze, wobei die Verschlusskappe in ein Luer-Lock-Gewinde geschraubt wird;
- Fig. 4: eine Abwandlung des Einsatzes gemäß Fig. 1 im Längsschnitt, wobei das Dichtungselement und das Druckelement relativ zueinander verdrehbar sind;
- Fig. 5: eine Aufsicht des Einsatzes gemäß Fig. 4;
- Fig. 6: eine praktische Anwendung des Einsatzes gemäß der Figuren 4 und 5 bei einer Spritze aus Glas mit einem drehmäßig verrastbaren Verschluss; und
- Fig. 7: eine weitere Abwandlung einer erfindungsgemäßen Spritze mit einem in einer Schließstellung verrastbaren Verschluss mit einer Schnappverbindung, der beim Lösen aus der Schließstellung zerstört wird und somit als Indikationselement für ein erstmaliges Öffnen des Verschlusses dient.

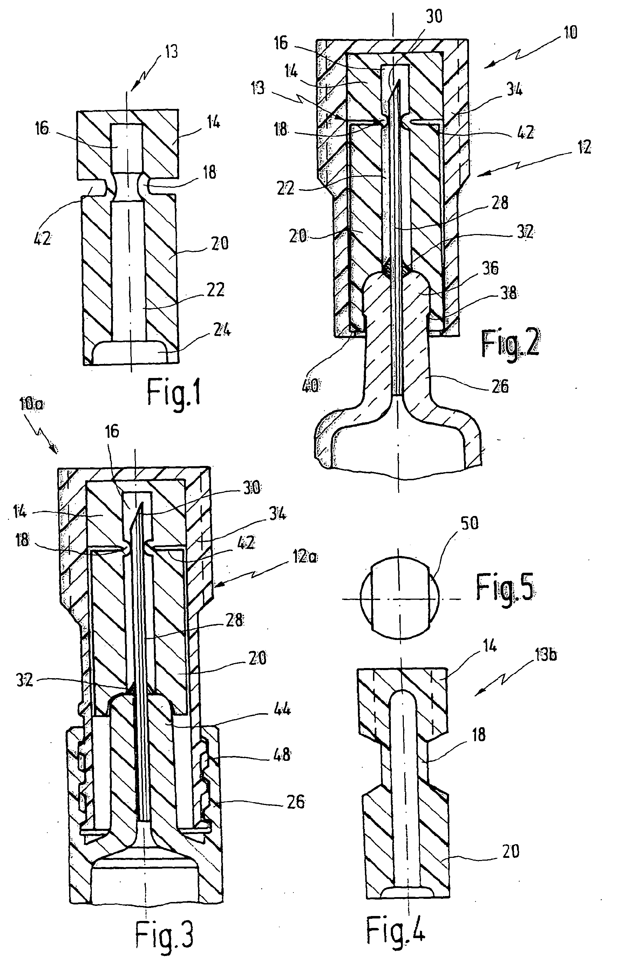

In Fig. 1 ist ein Einsatz 13 in längsgeschnittener Darstellung dargestellt, an dem zunächst ein mögliches Prinzip des erfindungsgemäßen Verschlusses erläutert werden soll.

Der Einsatz 13 besteht aus einem gummielastischen Material und weist ein Dichtungselement 14 und ein Druckelement 20 auf, die über einen ringförmigen, elastisch verformbaren Bereich 18 unter Bildung eines Spaltes 42 dazwischen miteinander verbunden sind. Das Dichtungselement 14 ist zylindrisch ausgebildet und weist einen zylindrischen Hohlraum 16 auf, der sich in einem entsprechenden zylindrischen Hohlraum 22 mit gleichem Durchmesser im Druckelement 20 fortsetzt. Der ringförmige, elastisch verformbare Bereich 18 stellt eine Durchmesserverjüngung zwischen den beiden Hohlräumen 16, 22 dar. Das Druckelement 20 weist einen etwas geringeren Außendurchmesser als das Dichtungselement 14 auf und ist an seinem unteren Ende durch eine Ausnehmung 24 mit größerem Durchmesser als der Hohlraum 22 abgeschlossen. Das Druckelement 20 kann mit der Ausnehmung 24 auf das distale Ende eines Spritzenkörpers aufgesetzt werden.

Unter "distal" wird in dieser Anmeldung die dem Patienten zugewandte bzw. dem Benutzer abgewandte Seite der Spritze verstanden.

Ein derartiger Einsatz 13 kann nun zur Abdichtung der Kanüle einer mit einer Flüssigkeit befüllten Spritze verwendet werden. Hierzu muss lediglich eine Relativbewegung zwischen dem Dichtungselement 14 und dem Druckelement 20 entweder durch eine Axialverschiebung oder durch ein Verdrehen zwischen diesen beiden Elementen erreicht werden, wodurch sich der Innendurchmesser des ringförmigen Bereiches 18 verringert und sich gleichzeitig die Größe des Spaltes 42 zwischen dem Dichtungselement 14 und dem Druckelement 20 verkleinert. Durch eine entsprechende Verringerung des Innendurchmessers des ringförmigen Bereiches 18 legt sich dieser an die Außenoberfläche einer Kanüle der Spritze an und kann diese flüssigkeitsdicht abdichten.

Auf diese Weise kann ein sicherer Verschluss einer mit einem Pharmazeutikum gefüllten Spritze aus Glas oder Kunststoff auf der distalen Seite erreicht werden, damit die enthaltene Flüssigkeit nicht austreten kann, auch nicht nach einer Lagerzeit von mehreren Jahren.

Zweckmäßigerweise ist der Außendurchmesser des Dichtungselementes 14 etwas größer als der Außendurchmesser des Druckelementes 20, um eine Manipulation des Dichtungselementes 14 durch eine Verschlusskappe des Verschlusses zu erleichtern, um die gewünschte Durchmesserverringerung herbeizuführen.

Eine erste mögliche praktische Ausführung einer erfindungsgemäßen Spritze ist in Fig. 2 dargestellt und insgesamt mit der Ziffer 10 bezeichnet.

Die Spritze 10 weist einen aus Glas bestehenden Spritzenkörper 26 auf, in dessen distales Ende eine Kanüle 28 mit einem Kleber 32 eingeklebt ist. Zum Verschluss der Spritze 10 am distalen Ende und zum Schutz der Kanüle 28 dient ein insgesamt mit 12 bezeichneter Verschluss. Der Verschluss 12 weist einen Einsatz 13 der zuvor anhand von Fig. 1 erläuterten Art mit einem Dichtungselement 14 und einem Druckelement 20 auf. Das Druckelement 20 ist an seiner, dem Spritzenkörper 26 zugewandten Seite mit einer geeigneten Ausnehmung versehen, so dass das Druckelement 20 auf einen entsprechend geformten Wulst 36 am Ende des Spritzenkörpers 26 aufgesetzt und hiermit verrastet werden kann. Zur Verrastung dient ein Vorsprung 38, der den Wulst 36 hintergreift.

Der Einsatz 13 ist von einer Verschlusskappe 34 umschlossen, mittels derer das Dichtungselement 14 etwas in Richtung auf das Druckelement 20 verschoben ist. In der in Fig. 2 gezeigten Schließstellung hintergreift so die Verschlusskappe 34 mit einem radial nach innen hervorstehenden Vorsprung 40 das proximale Ende des Druckelementes 20 und rastet daran ein. Auf diese Weise kann der Verschluss 12 in der Schließstellung am Wulst 36 des Spritzenkörpers 26 sicher verrastet werden.

Durch die Axialverschiebung des Dichtungselementes 14 in Richtung auf das Druckelement 20 ist der Spalt 42 zwischen Dichtungselement 14 und Druckelement 20 im Vergleich zu der Darstellung gemäß Fig. 1 deutlich verkleinert, wodurch der ringförmige, elastisch verformbare Bereich 18 sich auf die Außenoberfläche der Kanüle 28 dichtend anlegt. So erfolgt kurz hinter der Spitze 30 der Kanüle 28 durch den ringförmigen Bereich 18 eine flüssigkeitsdichte Abdichtung der Kanüle 28, so dass aus dem Lumen der Spritze und der Kanüle 28 keine Flüssigkeit nach außen gelangen kann.

Hierbei ist die Spitze 30 der Kanüle 28 im Hohlraum 16 des Dichtungselementes 14 berührungsfrei aufgenommen, so dass eine Beschädigung der Spitze 30 oder eine Kontamination durch Einstechen in das Material des Dichtungselementes 14 sicher vermieden wird. Zur Benutzung der Spritze 10 muss lediglich die Verschlusskappe 34 nach außen abgezogen und aus der Raststellung entfernt werden. Während der Abziehbewegung wird das Dichtungselement 14 nach außen bewegt, so dass sich der Spalt 42 zwischen Dichtungselement 14 und Druckelement 20 vergrößert und der Verschluss 12 leicht von der Kanüle 28 abgezogen werden kann.

Eine Abwandlung der Ausführung gemäß Fig. 2 ist in Fig. 3 dargestellt und insgesamt mit der Ziffer 10a bezeichnet.

Hierbei werden, wie auch bei den nachfolgenden Ausführungen, für entsprechende Teile entsprechende Bezugsziffern verwendet.

Bei der Spritze 10a handelt es sich um eine Kunststoffspritze, bei der die Verschlusskappe 34 mit einem Luer-Lock-Gewinde 48 in das distale Ende des Spritzenkörpers 26 eingeschraubt ist. Die Kanüle 28 ist in eine entsprechend geformte Ausnehmung am distalen Ende 44 des Spritzenkörpers 26 mittels eines Klebers 32 eingeklebt. Durch eine Drehbewegung der Verschlusskappe 34 kann beim Verschluss 12a das Dichtungselement 14 in Axialrichtung zum Druckelement 20 hin bewegt werden, um, wie zuvor anhand von Fig. 2 beschrieben, den Spalt 42 zwischen Dichtungselement 14 und Druckelement 20 zu verkleinern.

Wie bei der Ausführung gemäß Fig. 2 wird auf diese Weise der Innendurchmesser des ringförmigen Bereiches 18 verkleinert, so dass sich dieser kurz hinter der Spitze 30 der Kanüle 28 auf deren Außenoberfläche dichtend anlegt und diese in der in Fig. 3 dargestellten Schließstellung flüssigkeitsdicht abdichtet. Der Verschluss 12a kann leicht durch Aufschrauben des Luer-Lock-Gewindes aus der Schließstellung gelöst werden, so dass die Verschlusskappe 34 abgezogen werden kann, um die Spritze zu benutzen.

Eine weitere Variante des erfindungsgemäßen Verschlusses wird im Folgenden anhand der Figuren 4 bis 6 näher erläutert. Der Verschluss weist wiederum einen Einsatz 13b auf, der aus einem Dichtungselement 14 und einem Druckelement 20 besteht, die über einen ringförmigen, elastisch verformbaren Bereich 18 miteinander verbunden sind.

Im Unterschied zu den zuvor erläuterten Ausführungen kann hierbei eine Durchmesserverringerung des ringförmigen Bereiches 18 nicht durch ein Zusammendrücken, sondern durch ein Verdrehen erreicht werden. Indem das Dichtungselement 14 und das Druckelement 20 gegeneinander verdreht werden, ergibt sich eine Durchmesserverringerung des ringförmigen Bereiches 18, um eine Abdichtung einer zuvor eingeführten Kanüle in diesem Bereich zu erzielen. Wie aus Fig. 5 zu ersehen ist, kann das Dichtungselement 14 etwa an seinen beiden Außenflächen Abflachungen 50 aufweisen, um ein einfaches Verdrehen des Dichtungselementes 14 mit Hilfe einer aufgesetzten Verschlusskappe zu ermöglichen.

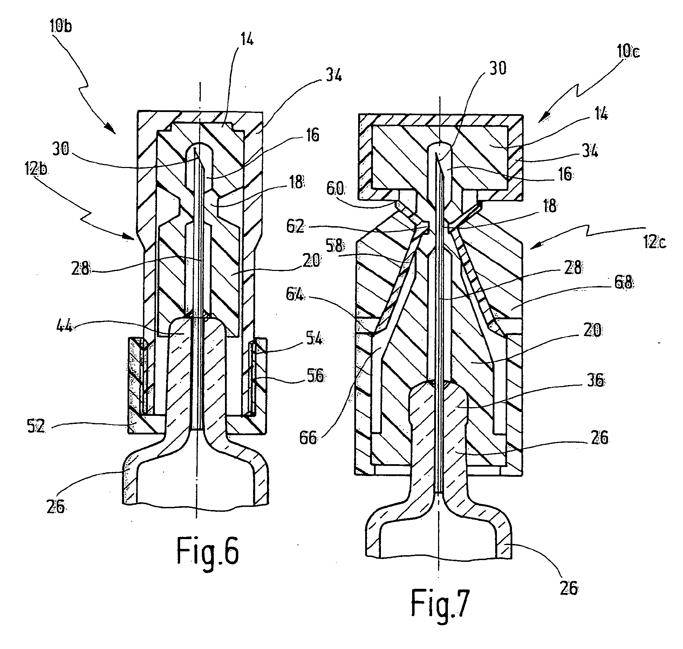

Eine derartige Ausführung einer solchen Spritze mit einem solchen Verschluss in Fig. 6 dargestellt und insgesamt mit 10b bezeichnet. Die Spritze 10b weist einen Verschluss 12b mit einer Verschlusskappe 34 auf, mittels derer das Dichtungselement 14 relativ zum Druckelement 20 verdreht werden kann. Dies führt zu einer Durchmesserverringerung des ringförmigen Bereiches 18, durch den das Dichtungselement 14 und das Druckelement 20 miteinander verbunden sind. So wird die Kanüle 28 wiederum in der Nähe ihrer Spitze 30 durch den ringförmigen Bereich 18 abgedichtet. Die Verschlusskappe 34 ist in der in Fig. 6 dargestellten Schließstellung mit geeigneten Rastelementen 56 an zugeordneten Rastelementen 54 eines auf dem distalen Ende 44 des Spritzenkörpers 26 gehaltenen Befestigungselement verrastet. Eine Sicherung gegen ein Abziehen nach außen kann im einfachsten Fall reibschlüssig gewährleistet werden oder etwadurch weitere Rastelemente (nicht dargestellt).

Eine weitere Ausführung einer erfindungsgemäßen Spritze 10c mit einem erfindungsgemäßen Verschluss 12c ist in Fig. 7 dargestellt.

Der Verschluss 12c weist hierbei wiederum ein Dichtungselement 14 und ein Druckelement 20 auf, die über einen elastisch verformbaren, ringförmigen Bereich 18 miteinander verbunden sind. Das Druckelement 20 ist an seinem proximalen Ende auf einen Wulst 36 des Spritzenkörpers 26 aufgesetzt und mit diesem verrastet. Über das Druckelement 20 ist ein Aufsatz 68 von außen aufgeschoben und am proximalen Ende des Druckelementes 20 mit diesem verrastet.

Die Verschlusskappe 34 umschließt das Dichtungselement 14 an seiner Außenseite und weist an ihrem proximalen Ende zwei Klappbügel 58 auf, die über Filmscharniere 60 an beiden Seiten mit der Verschlusskappe 34 verbunden sind und relativ zu dieser verschwenkbar sind. Diese Klappbügel 58 sind durch entsprechend geformte Ausnehmungen des Aufsatzes 68 hindurchgeführt und mit ihren Enden 64 in zugeordnete Schlitze 66 des Aufsatzes 68 eingerastet. Die Klappbügel 58 greifen in der in Fig. 7 dargestellten Schließstellung mit entsprechenden radialen Vorsprüngen 62 in den ringförmigen Bereich 18 von außen ein und pressen diesen auf die Außenoberfläche der Kanüle 28.

Die Verschlusskappe 34 ist in der in Fig. 7 gezeigten Schließstellung derart mit den Enden 64 in den Schlitzen 66 verrastet, dass ein Lösen durch ein Abziehen der Verschlusskappe 34 nach außen nur nach einer entsprechenden Zerstörung der Klappbügel 58 ermöglicht ist, was optisch erkennbar ist.

Auf diese Weise ist ein Originalitätsverschluss gewährleistet, da ein erstmaliges Abziehen der Verschlusskappe 34 aus der Schließstellung heraus zu einer Zerstörung der Klappbügel 58 im Bereich ihrer Enden 64 führt.

## Patentansprüche

1. Spritze mit einem Verschluss, mit einer aus einem Spritzenkörper (26) hervorstehenden Kanüle (28), deren Spitze (30) in einer Schließstellung der Spritze (10, 10a, 10b, 10c) von einer Verschlusskappe (34) umschlossen ist, und mit einem Dichtungselement (14) zur Abdichtung der Kanüle (28) in der Schließstellung, **dadurch gekennzeichnet, dass** das Dichtungselement (14) einen ringförmigen, elastisch verformbaren Bereich (18) aufweist, der mittels der Verschlusskappe (34) an die Außenoberfläche der Kanüle (28) dichtend anpressbar ist.

2. Spritze nach Anspruch 1, **dadurch gekennzeichnet, dass** das Dichtungselement (14) einen Hohlraum (16) aufweist, in dem die Spitze (30) der Kanüle (28) in der Schließstellung vorzugsweise berührungsfrei aufgenommen ist.

3. Spritze nach Anspruch 1 oder 2, **dadurch gekennzeichnet, dass** das Dichtungselement (14) über den ringförmigen Bereich (18) mit einem Druckelement (20) verbunden ist, und dass der ringförmige Bereich (18) durch eine Relativbewegung zwischen Dichtelement (14) und Druckelement (20) auf die Außenoberfläche der Kanüle (28) anpressbar ist.

4. Spritze nach Anspruch 3, **dadurch gekennzeichnet, dass** zumindest das Dichtungselement (14) von der Verschlusskappe zumindest teilweise umschlossen ist und mittels der Verschlusskappe (34) relativ zum Druckelement (20) bewegbar ist.

5. Spritze nach Anspruch 3 oder 4, **dadurch gekennzeichnet, dass** das Dichtungselement (14) und das Druckelement (20) axial zueinander beweglich sind.

6. Spritze nach Anspruch 3, 4 oder 5, **dadurch gekennzeichnet, dass** das Dichtungselement (14) und das Druckelement (20) gegeneinander verdrehbar sind.

7. Spritze nach einem der vorhergehenden Ansprüche, **dadurch gekennzeichnet, dass** die Verschlusskappe (34) in der Schließstellung verrastbar ist.

8. Spritze nach einem der vorhergehenden Ansprüche, **dadurch gekennzeichnet, dass** am distalen Ende des Spritzenkörpers (26) ein Wulst (36) vorgesehen ist, an dem die Verschlusskappe (34) oder das Druckelement (20) in der Schließstellung befestigt ist.

9. Spritze nach einem der vorhergehenden Ansprüche, **dadurch gekennzeichnet, dass** die Verschlusskappe (34) mittels eines Gewindes, vorzugsweise mittels eines Luer-Lock-Gewindes (48), am Spritzenkörper (26) festlegbar ist.

10. Spritze nach einem der vorhergehenden Ansprüche, **dadurch gekennzeichnet, dass** mindestens ein Indikatorelement vorgesehen ist, das eine erstmalige Bewegung der Verschlusskappe aus der Schließstellung anzeigt.

11. Spritze nach einem der vorhergehenden Ansprüche, **dadurch gekennzeichnet, dass** die Verschlusskappe (34) eine Schnappverbindung (58, 66) mit Bügelelementen (58) aufweist, die in der Schließstellung den ringförmigen Bereich (18) auf die Außenoberfläche der Kanüle (28) pressen.

12. Spritze nach Anspruch 11, **dadurch gekennzeichnet, dass** die Schnappverbindung (58, 66) als nicht zerstörungsfrei lösbare Schnappverbindung ausgebildet ist, bei der eine erstmalige Bewegung der Verschlusskappe (34) aus der Schließstellung optisch erkennbar ist.

13. Spritze nach einem der Ansprüche 3 bis 12, **dadurch gekennzeichnet, dass** Dichtungselement (14) und das Druckelement (20) aus demselben Material bestehen und einstückig ausgebildet sind.

14. Verschluss für eine Spritze, die eine aus einem Spritzenkörper (26) hervorstehende Kanüle (28) aufweist, wobei der Verschluss (12, 12a, 12b, 12c) zur Montage auf einem Ende des Spritzenkörpers (26) ausgebildet ist und eine Verschlusskappe (34) aufweist, die eine Spitze (30) der Kanüle (28) in einer Schließstellung umschließt, und mit einem Dichtungselement (14) zur Abdichtung der Kanüle (28) in der Schließstellung, **dadurch gekennzeichnet, dass** das Dichtungselement (14) einen ringförmigen, elastisch verformbaren Bereich (18) aufweist, der mittels der Verschlusskappe (34) an die Außenoberfläche der Kanüle (28) dichtend anpressbar ist.

15. Verschluss nach Anspruch 14, **dadurch gekennzeichnet, dass** das Dichtungselement (14) einen Hohlraum (16) aufweist, in dem eine Spitze (30) der Kanüle (28) in der Schließstellung vorzugsweise berührungsfrei aufgenommen ist.

16. Verschluss nach Anspruch 14 oder 15, **dadurch gekennzeichnet, dass** das Dichtungselement (14) über den ringförmigen Bereich (18) mit einem Druckelement (20) verbunden ist, und dass der ringförmige Bereich (18) durch eine Relativbewegung zwischen Dichtelement (14) und Druckelement (20) auf die Außenoberfläche der Kanüle (28) anpressbar ist.

17. Verschluss nach Anspruch 16, **dadurch gekennzeichnet, dass** zumindest das Dichtungselement (14) von der Verschlusskappe (34) zumindest teilweise umschlossen ist und mittels der Verschlusskappe (34) relativ zum Druckelement (20) bewegbar ist.

18. Verschluss nach Anspruch 16 oder 17, **dadurch gekennzeichnet, dass** das Dichtungselement (14) und das Druckelement (20) axial zueinander beweglich sind.

19. Verschluss nach Anspruch 16, 17 oder 18, **dadurch gekennzeichnet, dass** das Dichtungselement (14) und das Druckelement (20) gegeneinander verdrehbar sind.

20. Verschluss nach einem der Ansprüche 14 bis 19, **dadurch gekennzeichnet, dass** die Verschlusskappe (34) in der Schließstellung verrastbar ist.

21. Verschluss nach einem der Ansprüche 14 bis 20, **dadurch gekennzeichnet, dass** am distalen Ende des Spritzenkörpers (26) ein Wulst (36) vorgesehen ist, an dem die Verschlusskappe (34) oder das Druckelement (20) in der Schließstellung befestigt ist.

22. Verschluss nach einem der Ansprüche 14 bis 21, **dadurch gekennzeichnet, dass** die Verschlusskappe (34) mittels eines Gewindes, vorzugsweise mittels eines Luer-Lock-Gewindes (48) am Spritzenkörper (26) festlegbar ist.

23. Verschluss nach einem der Ansprüche 14 bis 22, **dadurch gekennzeichnet, dass** mindestens ein Indikatorelement vorgesehen ist, das eine erstmalige Bewegung der Verschlusskappe aus der Schließstellung anzeigt.

24. Verschluss nach einem der Ansprüche 14 bis 23, **dadurch gekennzeichnet, dass** die Verschlusskappe (34) eine Schnappverbindung (58, 66) mit Bügelelementen (58) aufweist, die in der Schließstellung den ringförmigen Bereich (18) auf die Außenoberfläche der Kanüle (28) pressen.

25. Verschluss nach Anspruch 24, **dadurch gekennzeichnet, dass** die Schnappverbindung (58, 66) als nicht zerstörungsfrei lösbare Schnappverbindung ausgebildet ist, bei der eine erstmalige Bewegung der Verschlusskappe (34) aus der Schließstellung optisch erkennbar ist.

26. Verschluss nach einem der Ansprüche 14 bis 25, **dadurch gekennzeichnet, dass** Dichtungselement (14) und das Druckelement (20) aus demselben Material bestehen und einstückig ausgebildet sind.
